(19) Europäisches Patentamt
European Patent Office
Office européen des brevets

(11) EP 1 327 639 A1

(12) EUROPEAN PATENT APPLICATION

(43) Date of publication:
**16.07.2003 Bulletin 2003/29**

(51) Int Cl.[7]: **C07K 14/47**, C12Q 1/68, A61K 38/17

(21) Application number: **02000253.1**

(22) Date of filing: **15.01.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **BAYER AG**
**51368 Leverkusen (DE)**

(72) Inventors:
- **Stropp, Udo, Dr.**
  **42781 Haan (DE)**
- **Schwers, Stephan, Dr.**
  **51145 Köln (DE)**
- **Kallabis, Harals, Dr.**
  **51061 Köln (DE)**
- **Schmitz, Gerd, Dr.**
  **93042 Regensburg (DE)**

# (54) Prediction of cardiovascular disease by genetic polymorphisms in cardiovascular associated genes

(57) The present invention relates to isolated polynucleotides encoding a cardiovascular associated (CA) gene polypeptide useful in methods to identify therapeutic agents and useful for preparation of a medicament to treat cardiovascular disease, the polynucleotide is selected from the group comprising:

SEQ ID 1, 2, 3, 4, 5, 6 with allelic variation as indicated in the sequences section contained in a functional surrounding like full length cDNA for CA gene polypeptide and with or without the CA gene promoter sequence.

The invention also provides diagnostic methods and kits including antibodies determining whether a human subject is at risk for a cardiovascular disease. The invention provides further polymorphic sequences and other genes.

EP 1 327 639 A1

## Description

### Technical Field

This invention relates to genetic polymorphisms useful for assessing cardiovascular risks in humans, including, but not limited to, atherosclerosis, ischemia/reperfusion, hypertension, restenosis, arterial inflammation, myocardial infarction, and stroke. Specifically, the present invention identifies and describes gene variations which are individually present in humans with cardiovascular disease states, relative to humans with normal, or non-cardiovascular disease states, and/or in response to medications relevant to cardiovascular disease. Further, the present invention provides methods for the identification and therapeutic use of compounds as treatments of cardiovascular disease. Moreover, the present invention provides methods for the diagnostic monitoring of patients undergoing clinical evaluation for the treatment of cardiovascular disease, and for monitoring the efficacy of compounds in clinical trials. Still further, the present invention provides methods to use gene variations to predict personal medication schemes omitting adverse drug reactions. Additionally, the present invention describes methods for the diagnostic evaluation and prognosis of various cardiovascular diseases, and for the identification of subjects exhibiting a predisposition to such conditions.

### Background of the Invention

Cardiovascular disease is a major health risk throughout the industrialized world.

Cardiovascular diseases include but are not limited by the following disorders of the heart and the vascular system: congestive heart failure, myocardial infarction, atherosclerosis, ischemic diseases of the heart, coronary heart disease, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others).

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis, the most prevalent of vascular diseases, is the principal cause of heart attack, stroke, and gangrene of the extremities, and thereby the principal cause of death. Atherosclerosis is a complex disease involving many cell types and molecular factors (for a detailed review, see Ross, 1993, Nature 362: 801-809 and Lusis, A. J., Nature 407, 233-241 (2000)). The process, in normal circumstances a protective response to insults to the endothelium and smooth muscle cells (SMCs) of the wall of the artery, consists of the formation of fibrofatty and fibrous lesions or plaques, preceded and accompanied by inflammation. The advanced lesions of atherosclerosis may occlude the artery concerned, and result from an excessive inflammatory-fibroproliferative response to numerous different forms of insult. For example, shear stresses are thought to be responsible for the frequent occurrence of atherosclerotic plaques in regions of the circulatory system where turbulent blood flow occurs, such as branch points and irregular structures.

The first observable event in the formation of an atherosclerotic plaque occurs when blood-borne monocytes adhere to the vascular endothelial layer and transmigrate through to the sub-endothelial space. Adjacent endothelial cells at the same time produce oxidized low density lipoprotein (LDL). These oxidized LDLs are then taken up in large amounts by the monocytes through scavenger receptors expressed on their surfaces. In contrast to the regulated pathway by which native LDL (nLDL) is taken up by nLDL specific receptors, the scavenger pathway of uptake is not regulated by the monocytes.

These lipid-filled monocytes are called foam cells, and are the major constituent of the fatty streak. Interactions between foam cells and the endothelial and SMCs which surround them lead to a state of chronic local inflammation which can eventually lead to smooth muscle cell proliferation and migration, and the formation of a fibrous plaque. Such plaques occlude the blood vessel concerned and thus restrict the flow of blood, resulting in ischemia.

Ischemia is a condition characterized by a lack of oxygen supply in tissues of organs due to inadequate perfusion. Such inadequate perfusion can have number of natural causes, including atherosclerotic or restenotic lesions, anemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply.

The most common cause of ischemia in the heart is atherosclerotic disease of epicardial coronary arteries. By reducing the lumen of these vessels, atherosclerosis causes an absolute decrease in myocardial perfusion in the basal state or limits appropriate increases in perfusion when the demand for flow is augmented. Coronary blood flow can also be limited by arterial thrombi, spasm, and, rarely, coronary emboli, as well as by ostial narrowing due to luetic aortitis. Congenital abnormalities, such as anomalous origin of the left anterior descending coronary artery from the pulmonary artery, may cause myocardial ischemia and infarction in infancy, but this cause is very rare in adults. Myocardial ischemia can also occur if myocardial oxygen demands are abnormally increased, as in severe ventricular hypertrophy due to hypertension or aortic stenosis. The latter can be present with angina that is indistinguishable from that caused by coronary atherosclerosis. A reduction in the oxygen-carrying capacity of the blood, as in extremely severe anemia or in the presence of carboxy-hemoglobin, is a rare cause of myocardial ischemia. Not infrequently, two or more causes of ischemia will coexist, such as an increase in oxygen demand due to left ventricular hypertrophy and a reduction in oxygen supply secondary to coronary atherosclerosis.

The foregoing studies are aimed at defining the role of particular gene variations presumed to be involved in the misleading of normal cellular function leading to cardiovascular disease. However, such approaches cannot identify the full panoply of gene variations that are involved in the disease process.

At present, the only available treatments for cardiovascular disorders are pharmaceutical based medications that are not targeted to an individual's actual defect; examples include angiotensin converting enzyme (ACE) inhibitors and diuretics for hypertension, insulin supplementation for non-insulin dependent diabetes mellitus (MDDM), cholesterol reduction strategies for dyslipidaemia, anti-coagulants, β blockers for cardiovascular disorders and weight reduction strategies for obesity. If targeted treatment strategies were available it might be possible to predict the response to a particular regime of therapy and could markedly increase the effectiveness of such treatment. Although targeted therapy requires accurate diagnostic tests for disease susceptibility, once these tests are developed the opportunity to utilize targeted therapy will become widespread. Such diagnostic tests could initially serve to identify individuals at most risk of hypertension and could allow them to make changes in lifestyle or diet that would serve as preventative measures. The benefits associated by coupling the diagnostic tests with a system of targeted therapy could include the reduction in dosage of administered drugs and thus the amount of unpleasant side effects suffered by an individual. In more severe cases a diagnostic test may suggest that earlier surgical intervention would be useful in preventing a further deterioration in condition.

It is an object of the invention to provide genetic diagnosis of predisposition or susceptibility for cardiovascular diseases. Another related object is to provide treatment to reduce or prevent or delay the onset of disease in those predisposed or susceptible to this disease. A further object is to provide means for carrying out this diagnosis.

Accordingly, a first aspect of the invention provides a method of diagnosis of disease in an individual, said method comprising determining one, various or all genotypes in said individual of the genes listed in the Examples.

In another aspect, the invention provides a method of identifying an individual predisposed or susceptible to a disease, said method comprising determining one, various or all genotypes in said individual of the genes listed in the Examples.

The invention is of advantage in that it enables diagnosis of a disease or of certain disease states via genetic analysis which can yield useable results before onset of disease symptoms, or before onset of severe symptoms. The invention is further of advantage in that it enables diagnosis of predisposition or susceptibility to a disease or of certain disease states via genetic analysis.

The invention may also be of use in confirming or corroborating the results of other diagnostic methods. The diagnosis of the invention may thus suitably be used either as an isolated technique or in combination with other methods and apparatus for diagnosis, in which latter case the invention provides a further test on which a diagnosis may be assessed.

The present invention stems from using allelic association as a method for genotyping individuals; allowing the investigation of the molecular genetic basis for cardiovascular diseases. In a specific embodiment the invention tests for the polymorphisms in the sequences of the listed genes in the Examples. The invention demonstrates a link between this polymorphisms and predispositions to cardiovascular diseases by showing that allele frequencies significantly differ when individuals with "bad" serum lipids are compared to individuals with "good" serum levels. The meaning of "good and bad" serum lipid levels is defined in Table 1.

Certain disease states would benefit, that is to say the suffering of the patient may be reduced or prevented or delayed, by administration of treatment or therapy in advance of disease appearance; this can be more reliably carried out if advance diagnosis of predisposition or susceptibility to disease can be diagnosed.

Detailed Description of the Invention

The present invention is based at least in part on the discovery that a specific allele of a polymorphic region of a so called "candidate gene" is associated with CVD.

"Candidate gene" as used herein includes genes that can be assigned to either normal cardiovascular function or to metabolic pathways that are related to onset and/or progression of cardiovascular diseases. As the development of cardiovascular diseases is not completely understood, the term "candidate gene" may also comprise genes with presently unknown function.

For the present invention the following candidate genes were analyzed:

- Genes found to be expressed in cardiac tissue (Hwang et al., Circulation 1997, 96:4146-4203).
- Genes from the following metabolic pathways and their regulatory elements:

**Lipid metabolism**

Numerous studies have shown a connection between serum lipid levels and cardiovascular diseases. Candidate genes falling into this group include but are not limited by genes of the cholesterol pathway, apolipoproteins and their modifiying factors.

**Coagulation**

Ischemic diseases of the heart and in particular myocardial infarction may be caused by a thrombotic occlusion. Genes falling into this group include all genes of the coagulation cascade and their regulatory elements.

**Inflammation**

Complications of atherosclerosis are the most common causes of death in Western societies. In broad outline atherosclerosis can be considered to be a form of chronic inflammation resulting from interaction modified lipoproteins, monocyte-derived macrophages,T cells, and the normal cellular elements of the arterial wall. This inflammatory process can ultimately lead to the development of complex lesions, or plaques, that protrude into the arterial lumen. Finally plaque rupture and thrombosis result in the acute clinical complications of myocardial infarction and stroke (Glass et al., Cell 2001, 104:503-516).

It follows that all genes related to inflammatory processes, including but not limited by cytokines, cytokine receptors and cell adhesion molecules are candidate genes for CVD.

**Glucose and energy metabolism**

As glucose and energy metabolism is interdependent with the metabolism of lipids (see above) also the former pathways contain candidate genes. Energy metabolism in general also relates to obesity, which is an independent risk factor for CVD (Melanson et al., Cardiol Rev 2001 9:202-207). In addition high blood glucose levels are associated with many microvascular and macrovascular complications and may therefore affect an individuals disposition to CVD (Duckworth, Curr Atheroscler Rep 2001, 3:383-391).

**Hypertension**

As hypertension is an independent risk factor for CVD, also genes that are involved in the regulation of systolic and diastolic blood pressure affect an individuals risk for CVD (Safar, Curr Opin Cardiol 2000, 15:258-263). Interestingly hypertension and diabetes (see above) appear to be interdependent, since hypertension is approximately twice as frequent in patients with diabetes compared with patients without the disease. Conversely, recent data suggest that hypertensive persons are more predisposed to the development of diabetes than are normotensive persons (Sowers et al., Hypertension 2001, 37:1053-1059).

**Unclassified genes**

As stated above, the mechanisms that lead to cardiovascular diseases are not completely elucidated. Hence also candidate genes were analysed, which could not be assigned to the above listed categories. The present invention is based at least in part on the discovery of polymorphisms, that lie in genomic regions of unknown physiological function.

**Results**

After conducting an association study, we surprisingly found polymorphic sites in a number of candidate genes which show a strong correlation between healthy and CVD prone individuals. "Healthy" as used herein refers to individuals that neither suffer from existing CVD, nor exihibit an increased risk for CVD through their serum lipid level profile. "CVD prone" as used herein refers to individuals with existing CVD and/or a serum lipid profile that confers a high risk to get CVD (see Table 1 for definitions of healthy and CVD prone serum lipid levels). Polymorphic sites in candidate genes that were found to be significantly associated with "healthy" or "CVD prone" phenotypes will be refered to as "CVD associated SNPs" (CA SNPs). The respective genomic loci that harbour CA SNPs will be refered to as "CVD associated genes" (CA genes), irrespective of the actual function of this gene locus.

In particular we surprisingly found CA SNPs in the following genes:

**Apolipoprotein M (ApoM)**

Apolipoprotein M is a novel human apolipoprotein. The unique N-terminal amino acid sequence of apoM was found in an approximately 26-kDa protein present in a protein extract of triglyceride-rich lipoproteins (TGRLP). The isolated apoM cDNA (734 base pairs) encoded a 188-amino acid residue-long protein, distantly related to the lipocalin family. The mRNA of apoM was detected in the liver and kidney. Western blotting demonstrated apoM to be present in high density lipoprotein (HDL) and to a lesser extent in TGRLP and low density lipoproteins (LDL) (Xu, N.; Dahlbäck, B. 1999, J. Biol. Chem. 274: 31286-31290).

**Plasminogen activator inhibitor I (PAI1)**

Plasminogen activator inhibitor I regulates fibrinolysis; member of the serpin family of serine protease inhibitors.

Carmeliet et al. (1997, Circulation 96: 3180-3191) provided direct evidence to support a role for PAI1 in arterial wound healing. Techniques of arterial injury were designed in mice to model the histologic changes seen in human arteries following angioplasty and surgical anastomosis. PAI1 gene knockout mice underwent arterial injury followed by adenovirus-mediated PAI1 gene transfer by intravenous injection. Recombinant PAI1 expression was demonstrated in injured arteries and was found to inhibit neointima formation by inhibiting smooth muscle cell migration. Carmeliet et al. (1997, Circulation 96: 3180-3191) suggested that this may have implications for the treatment of arterial stenosis in humans following surgical intervention.

**Granulocyte-macrophage colony stimulating factor (GM-CSF)**

Granulocyte-macrophage colony stimulating factor regulates hematopoietic cell differentiation, gene expression, and cell growth.

**Oncostatin M (OSM)**

Oncostatin M is a member of the IL-6 family of cytokines that is primarily known for its effects on cell growth. In exploration of the potential of oncostatin M as an inflammatory effector, Modur et al. (1997, J. Clin. Invest. 100: 158-168) found that oncostatin M caused an acute inflammatory reaction in vivo and that it colocalized with TNF-secreting cells in chronically inflamed human vascular tissue. It induced transmigration of human neutrophils through monolayers of endothelial cells by stimulating the expression of adhesion molecules and chemokines. The pattern of endothelial cell responses, however, significantly differed from the response to TNF. Modur et al. (1997) concluded that oncostatin M, but not other IL-6 family members, fulfilled Koch's postulates as an inflammatory mediator. Since its effects on endothelial cells differ significantly from established mediators like TNF-alpha, it may uniquely contribute to the inflammatory cycle.

**Platelet-derived growth factor beta polypeptide (PDGFB)**

The protein encoded by this gene is a member of the platelet-derived growth factor family. The four members of this family are mitogenic factors for cells of mesenchymal origin and are characterized by a motif of eight cysteines. This gene product can exist either as a homodimer or as a heterodimer with the platelet-derived growth factor alpha polypeptide, where the dimers are connected by disulfide bonds. Mutations in this gene are associated with meningioma. Reciprocal translocations between chromosomes 22 and 7, at sites where this gene and that for COL1A1 are located, are associated with a particular type of skin tumor called dermatofibrosarcoma protuberans resulting from unregulated expression of growth factor. Two splice variants have been identified for this gene.
Disruption of the PDGFB gene or the gene for its receptor in mice leads to the development of lethal hemorrhage and edema in late embryogenesis and absence of kidney glomerular mesangial cells. Lindahl et al. (1997, Science 277: 242-245) found that mouse embryos deficient in PDGFB lack microvascular pericytes, which normally form part of the capillary wall, and develop numerous capillary microaneurysms that rupture at late gestation. Endothelial cells of the sprouting capillaries in mutant mice appeared to be unable to attract PDGF-receptor-beta-positive pericyte progenitor cells. Pericytes may contribute to the mechanical stability of the capillary wall. Comparisons made between PDGF-null mouse phenotypes suggested a general role for PDGFs in the development of myofibroblasts.

**Hepatocyte nuclear factor 4, alpha (HFN4A)**

Nuclear hormone receptor transcription factor; regulates liver specific gene expression
During the course of a search for susceptibility genes contributing to late-onset noninsulin-dependent diabetes mellitus (NIDDM), Zouali et al. (1997, Hum. Molec. Genet, 6: 1401-1408) found a suggestion for linkage with markers in the region of the HNF4A/MODY1 gene in a subset of French families with age at onset less than 45 years. This prompted Hani et al. (1998, J. Clin. Invest. 101: 521-526) to screen the HNF4A gene for mutations in 19 French NIDDM families diagnosed before 45 years of age. In 1 family they found a va1393-to-ile substitution. This mutation cosegregated with diabetes and impaired insulin secretion. Expression studies showed that the substitution was associated with a marked reduction of transactivation activity, a result consistent with this mutation contributing to the insulin secretory defect observed in the family.
As SNPs are linked to other SNPs in neighboring genes on a chromosome (Linkage Disequilibrium) those SNPs could also be used as marker SNPs. In a recent publication it was shown that SNPs are linked over 100 kb in some cases more than 150 kb (Reich D.E. et al. Nature 411, 199-204, 2001). Hence SNPs lying in regions neighbouring CA SNPs could be linked to the latter and by this being a CVD marker. These associations could be performed as described for the gene polymorphism in methods.

**Definitions**

For convenience, the meaning of certain terms and phrases employed in the specification, examples, and appended claims are provided below. Moreover, the definitions by itself are intended to explain a further background of the invention.
The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.
The term "allelic variant of a polymorphic region of a gene" refers to a region of a gene having one of several nucleotide sequences found in that region of the gene in other individuals.
"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.
The term "a homologue of a nucleic acid" refers to a nucleic acid having a nucleotide sequence having a certain degree of homology with the nucleotide sequence of the nucleic acid or complement thereof. A homologue of a double stranded nucleic acid having SEQ ID NO. X is intended to include nucleic acids having a nucleotide sequence which has a certain degree of homology with SEQ ID NO. X or with the complement thereof. Preferred homologous of nucleic acids are capable of hybridizing to the nucleic acid or complement thereof.

The term "interact" as used herein is meant to include detectable interactions between molecules, such as can be detected using, for example, a hybridization assay.
The term interact is also meant to include "binding" interactions between molecules. Interactions may be, for example, protein-protein, protein-nucleic acid, protein-small molecule or small molecule-nucleic acid in nature.

The term "intronic sequence" or "intronic nucleotide sequence" refers to the nucleotide sequence of an intron or portion thereof.

The term "isolated" as used herein with respect to nucleic acids, such as DNA or RNA, refers to molecules separated from other DNAs or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized.

Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "lipid" shall refer to a fat or fat-like substance that is insoluble in polar solvents such as water. The term "lipid" is intended to include true fats (e.g. esters of fatty acids and glycerol); lipids (phospholipids, cerebrosides, waxes); sterols (cholesterol, ergosterol) and lipoproteins (e.g. HDL, LDL and VLDL).

The term "locus" refers to a specific position in a chromosome. For example, a locus of a gene refers to the chromosomal position of the gene.

The term "modulation" as used herein refers to both up-regulation, (i.e., activation or stimulation), for example by agonizing, and down-regulation (i.e. inhibition or suppression), for example by antagonizing of a bioactivity (e.g. expression of a gene).

The term "molecular structure" of a gene or a portion thereof refers to the structure as defined by the nucleotide content (including deletions, substitutions, additions of one or more nucleotides), the nucleotide sequence, the state of methylation, and/or any other modification of the gene or portion thereof.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subject, the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, including peptide nucleic acids (PNA), morpholino oligonucleotides (J. Summerton and D. Weller, Antisense and Nucleic Acid Drug Development 7:187 (1997)) and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the term "adenosine", "cytidine", "guanosine", and "thymidine" are used. It is understood that if the nucleic acid is RNA, a nucleotide having a uracil base is uridine.

The term "nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction. The term "complement" and "reverse complement" are used interchangeably herein.

The term "operably linked" is intended to mean that the promoter is associated with the nucleic acid in such a manner as to facilitate transcription of the nucleic acid.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

To describe a "polymorphic site" in a nucleotide sequence often there is used an "ambiguity code" that stands for the possible variations of nucleotides in one site. The list of ambiguity codes is summarized in the following table:

| Ambiguity Codes (IUPAC Nomenclature) | |
|---|---|
| B | c/g/t |
| D | a/g/t |
| H | a/c/t |
| K | g/t |
| M | a/c |
| N | a/c/g/t |
| R | a/g |
| S | c/g |
| V | a/c/g |
| W | a/t |
| Y | c/t |

So, for example, a "R" in a nucleotide sequence means that either an "a" or a "g" could be at that position.

The terms "protein", "polypeptide" and "peptide" are used interchangeably herein when referring to a gene product.

A "regulatory element", also termed herein "regulatory sequence is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Thus, it is possible that genes have regulatory elements located in introns, exons, coding regions, and 3' flanking sequences. Such regulatory elements are also intended to be encompassed by the present invention and can be identified by any of the assays that can be used to identify regulatory elements in 5' flanking regions of genes.

The term "regulatory element" further encompasses "tissue specific" regulatory elements, i.e., regulatory elements which effect expression of the selected DNA sequence preferentially in specific cells (e.g., cells of a specific tissue). gene expression occurs preferentially in a specific cell if expression in this cell type is significantly higher than expression in other cell types. The term "regulatory element" also encompasses non-tissue specific regulatory elements, i.e., regulatory elements which are active in most cell types. Furthermore, a regulatory element can be a constitutive regulatory element, i.e., a regulatory element which constitutively regulates transcription, as opposed to a regulatory element which is inducible, i.e., a regulatory element which is active primarily in response to a stimulus. A stimulus can be, e.g., a molecule, such as a hormone, cytokine, heavy metal, phorbol ester, cyclic AMP (cAMP), or retinoic acid.

Regulatory elements are typically bound by proteins, e.g., transcription factors. The term "transcription factor" is intended to include proteins or modified forms thereof, which interact preferentially with specific nucleic acid sequences, i.e., regulatory elements, and which in appropriate conditions stimulate or repress transcription. Some transcription factors are active when they are in the form of a monomer. Alternatively, other transcription factors are active in the form of a dimer consisting of two identical proteins or different proteins (heterodimer). Modified forms of transcription factors are intended to refer to transcription factors having a post-translational modification, such as the attachment of a phosphate group. The activity of a transcription factor is frequently modulated by a post-translational modification. For example, certain transcription factors are active only if they are phosphorylated on specific residues. Alternatively, transcription factors can be active in the absence of phosphorylated residues and become inactivated by phosphorylation. A list of known transcription factors and their DNA binding site can be found, e.g., in public databases, e.g., TFMATRIX Transcription Factor Binding Site Profile database.

As used herein, the term "specifically hybridizes" or "specifically detects" refers to the ability of a nucleic acid molecule of the invention to hybridize to at least approximately 6, 12, 20, 30, 40, 50, 60, 70, 80, 90, 100, 110, 120, 130 or 140 consecutive nucleotides of either strand of a gene.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

"Candidate gene" as used herein includes genes that can be assigned to either normal cardiovascular function or to metabolic pathways that are related to onset and/or progression of cardiovascular diseases. As the development of cardiovascular diseases is not completely understood, the term "candidate gene" may also comprise genes with presently unknown function.

"CA SNP" (cardiovascular disease associated SNP) refers to a polymorphic site which shows a significant association with the risk to develop cardiovascular diseases.

"CA gene" (cardiovascular disease associated gene) refers to a genomic locus harbouring a CA SNP, irrespective of the actual function of this gene locus.

CA gene polypeptide refers to a polypeptide encoded at least in part by a CA gene

Methods for Assessing Cardiovascular Status

The present invention provides diagnostic methods for assessing cardiovascular status in a human individual. Cardiovascular status as used herein refers to the physiological status of an individual's cardiovascular system as reflected in one or more markers or indicators. Status markers include without limitation clinical measurements such as, e.g., blood pressure, electrocardiographic profile, and differentiated blood flow analysis as well as measurements of LDL- and HDL-Cholesterol levels, other lipids and other well established clinical parameters that are standard in the art. Status markers according to the invention include diagnoses of one or more cardiovascular syndromes, such as, e.g., hypertension, acute myocardial infarction, silent myocardial infarction, stroke, and atherosclerosis. It will be understood that a diagnosis of a cardiovascular syndrome made by a medical practitioner encompasses clinical measurements and medical judgement. Status markers according to the invention are assessed using conventional methods well known in the art. Also included in the evaluation of cardiovascular status are quantitative or qualitative changes in status markers with time, such as would be used, e.g., in the determination of an individual's response to a particular therapeutic regimen.

The methods are carried out by the steps of:

(i) determining the sequence of one or more polymorphic positions within one, sseveral or all of the genes listed in Examples or other genes mentioned in this file in the individual to establish a polymorphic pattern for the individual; and

(ii) comparing the polymorphic pattern established in (i) with the polymorphic patterns of humans exhibiting different markers of cardiovascular status. The polymorphic pattern of the individual is, preferably, highly similar and, most preferably, identical to the polymorphic pattern of individuals who exhibit particular status markers, cardiovascular syndromes, and/or particular patterns of response to therapeutic interventions. Polymorphic patterns may also include polymorphic positions in other genes which are shown, in combination with one or more polymorphic positions in the genes listed in the Examples, to correlate with the presence of particular status markers. In one embodiment, the method involves comparing an individual's polymorphic pattern with polymorphic patterns of individuals who have been shown to respond positively or negatively to a particular therapeutic regimen. Therapeutic regimen as used herein refers to treatments aimed at the elimination or amelioration of symptoms and events associated cardiovascular disease. Such treatments include without limitation one or more of alteration in diet, lifestyle, and exercise regimen; invasive and noninvasive surgical techniques such as atherectomy, angioplasty, and coronary bypass surgery; and pharmaceutical interventions, such as administration of ACE inhibitors, angiotensin II receptor antagonists, diuretics, alpha-adrenoreceptor antagonists, cardiac glycosides, phosphodiesterase inhibitors, beta-adrenoreceptor antagonists, calcium channel blockers, HMG-CoA reductase inhibitors, imidazoline receptor blockers, endothelin receptor blockers, organic nitrites, and modulators of protein function of genes listed in the Examples. Interventions with pharmaceutical agents not yet known whose activity correlates with particular polymorphic patterns associated with cardiovascular disease are also encompassed. It is contemplated, for example, that patients who are candidates for a particular therapeutic regimen will be screened for polymorphic patterns that correlate with responsivity to that particular regimen.

In a preferred embodiment, the method involves comparing an individual's polymorphic pattern with polymorphic patterns of individuals who exhibit or have exhibited one or more markers of cardiovascular disease, such as, e.g., elevated LDL-Cholesterol levels, high blood pressure, abnormal electrocardiographic profile, myocardial infarction, stroke, or atherosclerosis.

In practicing the methods of the invention, an individual's polymorphic pattern can be established by obtaining DNA from the individual and determining the sequence at predetermined polymorphic positions in the genes such as those described in this file.

The DNA may be obtained from any cell source. Non-limiting examples of cell sources available in clinical practice include blood cells, buccal cells, cervicovaginal cells, epithelial cells from urine, fetal cells, or any cells present in tissue obtained by biopsy. Cells may also be obtained from body fluids, including without limitation blood, saliva, sweat, urine, cerebrospinal fluid, feces, and tissue exudates at the site of infection or inflammation. DNA is extracted from the cell source or body fluid using any of the numerous methods that are standard in the art. It will be understood that the particular method used to extract DNA will depend on the nature of the source.

**Diagnostic and Prognostic Assays**

The present invention provides methods for determining the molecular structure of at least one polymorphic region of a gene, specific allelic variants of said polymorphic region being associated with cardiovascular disease. In one embodiment, determining the molecular structure of a polymorphic region of a gene comprises determining the identity of the allelic variant. A polymorphic region of a gene, of which specific alleles are associated with cardiovascular disease can be located in an exon, an intron, at an intron/exon border, or in the promoter of the gene.

The invention provides methods for determining whether a subject has, or is at risk, of developing a cardiovascular disease. Such disorders can be associated with an aberrant gene activity, e.g., abnormal binding to a form of a lipid, or an aberrant gene protein level. An aberrant gene protein level can result from an aberrant transcription or post-transcriptional regulation. Thus, allelic differences in specific regions of a gene can result in differences of gene protein due to differences in regulation of expression. In particular, some of the identified polymorphisms in the human gene may be associated with differences in the level of transcription, RNA maturation, splicing, or translation of the gene or transcription product.

In preferred embodiments, the methods of the invention can be characterized as comprising detecting, in a sample of cells from the subject, the presence or absence of a specific allelic variant of one or more polymorphic regions of a gene. The allelic differences can be: (i) a difference in the identity of at least one nucleotide or (ii) a difference in the number of nucleotides, which difference can be a single nucleotide or several nucleotides.

A preferred detection method is allele specific hybridization using probes overlapping the polymorphic site and having about 5, 10, 20, 25, or 30 nucleotides around the polymorphic region. Examples of probes for detecting specific allelic variants of the polymorphic region located in intron X are probes comprising a nucleotide sequence set forth in any of SEQ ID NO. X. In a preferred embodiment of the invention, several probes capable of hybridizing specifically to allelic variants are attached to a solid phase support, e.g., a "chip". Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (1996) Human Mutation 7:244 and in Kozal et al. (1996) Nature Medicine 2:753. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment. For example, the identity of the allelic variant of the nucleotide polymorphism of nucleotide A or C at position 227 of Seq ID 1 (baySNP2281) and that of other possible polymorphic regions can be determined in a single hybridization experiment.

In other detection methods, it is necessary to first amplify at least a portion of a gene prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 40 and 350 base pairs apart. Preferred primers for amplifying gene fragments of genes of this file are listed in Table 2 in the Examples.

Alternative amplification methods include: self sustained sequence replication (Guatelli, J. C. et al., 1990, Proc. Natl. Acad. Sci. U.S.A. 87:1874-1878), transcriptional amplification system (Kwoh, D. Y. et al., 1989, Proc. Natl. Acad. Sci. U.S.A. 86:1173-1177), Q-Beta Replicase (Lizardi, P. M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In one embodiment, any of a variety of sequencing reactions known in the art can be used to directly sequence at least a portion of a gene and detect allelic variants, e.g., mutations, by comparing the sequence of the sample sequence with the corresponding wild-type (control) sequence. Exemplary sequencing reactions include those based on techniques developed by Maxam and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including sequencing by mass spectrometry (see, for example, U.S. Pat. No. 5,547,835 and international patent application Publication Number WO 94/16101, entitled DNA Sequencing by Mass Spectrometry by H. Koster; U.S. Pat. No. 5,547,835 and international patent application Publication Number WO 94/21822 entitled "DNA Sequencing by Mass Spectrometry Via Exonuclease Degradation" by H. Koster), and U.S. Pat. No. 5,605,798 and International Patent Application No. PCT/US96/03651 entitled DNA Diagnostics Based on Mass Spectrometry by H. Koster; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleotide is detected, can be carried out.

Yet other sequencing methods are disclosed, e.g., in U.S. Pat. No. 5,580,732 entitled "Method of DNA sequencing employing a mixed DNA-polymer chain probe" and U.S. Pat. No. 5,571,676 entitled "Method for mismatch-directed in vitro DNA sequencing".

In some cases, the presence of a specific allele of a gene in DNA from a subject can be shown by restriction enzyme analysis. For example, a specific nucleotide polymorphism can result in a nucleotide sequence comprising a restriction site which is absent from the nucleotide sequence of another allelic variant.

In other embodiments, alterations in electrophoretic mobility is used to identify the type of gene allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the identity of an allelic variant of a polymorphic region is obtained by analyzing the movement of a nucleic acid comprising the polymorphic region in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used. Oligonucleotides used as primers for specific amplification may carry the allelic variant of interest in the center of the molecule (so that amplification depends on differential hybridization) (Gibbs et al (1989) Nucleic Acids Res. 17: 2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension. In addition it may be desirable to introduce a novel restriction site in the region of the mutation to create cleavage-based detection (Gasparini et al (1992) Mol. Cell Probes 6:1).

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a gene. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996)Nucleic Acids Res 24: 3728), OLA combined with PCR permits typing of two alleles in a single microtiter well. By marking each of the allele-specific primers with a unique hapten, i.e digoxigenin and fluorescein, each LA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA TM is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990), Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA TM in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For determining the identity of the allelic variant of a polymorphic region located in the coding region of a gene, yet other methods than those described above can be used. For example, identification of an allelic variant which encodes a mutated gene protein can be performed by using an antibody specifically recognizing the mutant protein in, e.g., immunohistochemistry or immunoprecipitation. Antibodies to wild-type gene protein are described, e.g., in Acton et al. (1999) Science 271:518 (antimouse gene antibody cross-reactive with human gene). Other antibodies to wild-type gene or mutated forms of gene proteins can be prepared according to methods known in the art. Alternatively, one can also measure an activity of an gene protein, such as binding to a lipid or lipoprotein. Binding assays are known in the art and involve, e.g., obtaining cells from a subject, and performing binding experiments with a labeled lipid, to determine whether binding to the mutated form of the receptor differs from binding to the wild-type of the receptor.

If a polymorphic region is located in an exon, either in a coding or non-coding region of the gene, the identity of the allelic variant can be determined by determining the molecular structure of the mRNA, pre-mRNA, or cDNA. The molecular structure can be determined using any of the above described methods for determining the molecular structure of the genomic DNA, e.g., sequencing and SSCP.

The methods described herein may be performed, for example, by utilizing prepackaged diagnostic kits, such as those described above, comprising at least one probe or primer nucleic acid described herein, which may be conveniently used, e.g., to determine whether a subject has or is at risk of developing a disease associated with a specific gene allelic variant.

Sample nucleic acid for using in the above-described diagnostic and prognostic methods can be obtained from any cell type or tissue of a subject. For example, a subject's bodily fluid (e.g. blood) can be obtained by known techniques (e.g. venipuncture) or from human tissues like heart (biopsies, transplanted organs). Alternatively, nucleic acid tests can be performed on dry samples (e.g. hair or skin). Fetal nucleic acid samples for prenatal diagnostics can be obtained from maternal blood as described in International Patent Application No.WO91/07660 to Bianchi.

Alternatively, amniocytes or chorionic villi may be obtained for performing prenatal testing.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, New York).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

In practicing the present invention, the distribution of polymorphic patterns in a large number of individuals exhibiting particular markers of cardiovascular status is determined by any of the methods described above, and compared with the distribution of polymorphic patterns in patients that have been matched for age, ethnic origin, and/or any other statistically or medically relevant parameters, who exhibit quantitatively or qualitatively different status markers. Correlations are achieved using any method known in the art, including nominal logistic regression, chi square tests or standard least squares regression analysis. In this manner, it is possible to establish statistically significant correlations between particular polymorphic patterns and particular cardiovascular statuses (given in p values). It is further possible to establish statistically significant correlations between particular polymorphic patterns and changes in cardiovascular status such as, would result, e.g., from particular treatment regimens. In this manner, it is possible to correlate polymorphic patterns with responsivity to particular treatments.

## Isolated Polymorphic Nucleic Acids, Probes, and Vectors

The present invention provides isolated nucleic acids comprising the polymorphic positions described herein for human genes; vectors comprising the nucleic acids; and transformed host cells comprising the vectors. The invention also provides probes which are useful for detecting these polymorphisms.

In practicing the present invention, many conventional techniques in molecular biology, microbiology, and recombinant DNA, are used. Such techniques are well known and are explained fully in, for example, Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D. N. Glover ed.); Oligonucleotide Synthesis, 1984, (M. L.Gait ed.); Nucleic Acid Hybridization, 1985, (Hames and Higgins); Ausubel et al., Current Protocols in Molecular Biology, 1997, (John Wiley and Sons); and Methods in Enzymology Vol. 154 and Vol. 155 (Wu and Grossman, and Wu, eds., respectively).

Insertion of nucleic acids (typically DNAs) comprising the sequences in a functional surrounding like full length cDNA of the present invention into a vector is easily accomplished when the termini of both the DNAs and the vector comprise compatible restriction sites. If this cannot be done, it may be necessary to modify the termini of the DNAs and/or vector by digesting back single-stranded DNA overhangs generated by restriction endonuclease cleavage to produce blunt ends, or to achieve the same result by filling in the single-stranded termini with an appropriate DNA polymerase.

Alternatively, any site desired may be produced, e.g., by ligating nucleotide sequences (linkers) onto the termini. Such linkers may comprise specific oligonucleotide sequences that define desired restriction sites. Restriction sites can also be generated by the use of the polymerase chain reaction (PCR). See, e.g., Saiki et al., 1988, Science 239:48. The cleaved vector and the DNA fragments may also be modified if required by homopolymeric tailing.

The nucleic acids may be isolated directly from cells or may be chemically synthesized using known methods. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

The nucleic acids of the present invention may be flanked by native gene sequences, or may be associated with heterologous sequences, including promoters, enhancers, response elements, signal sequences, polyadenylation sequences, introns, 5'- and 3'-noncoding regions, and the like. The nucleic acids may also be modified by many means known in the art. Non-limiting examples of such modifications include methylation, "caps", substitution of one or more of the naturally occurring nucleotides with an analog, internucleotide modifications such as, for example, those with uncharged linkages (e.g., methyl phosphonates, phosphotriesters, phosphoroamidates, carbamates, morpholines etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also included. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the gene sequences or derivatives or fragments thereof of genes described in the Examles. A large number of vectors, including plasmid and fungal vectors, have been described for replication and/or expression in a variety of eukaryotic and prokaryotic hosts, and may be used for gene therapy as well as for simple cloning or protein expression. Non-limiting examples of suitable vectors include without limitation pUC plasmids, pET plasmids (Novagen, Inc., Madison, Wis.), or pRSET or pREP (Invitrogen, San Diego, Calif.), and many appropriate host cells, using methods disclosed or cited herein or otherwise known to those skilled in the relevant art. The particular choice of vector/host is not critical to the practice of the invention.

Suitable host cells may be transformed/transfected/infected as appropriate by any suitable method including electroporation, $CaCl_2$ mediated DNA uptake, fungal or viral infection, microinjection, microprojectile, or other established methods. Appropriate host cells included bacteria, archebacteria, fungi, especially yeast, and plant and animal cells, especially mammalian cells. A large number of transcription initiation and termination regulatory regions have been isolated and shown to be effective in the transcription and translation of heterologous proteins in the various hosts. Examples of these regions, methods of isolation, manner of manipulation, etc. are known in the art. Under appropriate expression conditions, host cells can be used as a source of recombinantly produced peptides and polypeptides encoded by genes of the Examples. Nucleic acids encoding peptides or polypeptides from gene sequences of the Examples may also be introduced into cells by recombination events. For example, such a sequence can be introduced into a cell and thereby effect homologous recombination at the site of an endogenous gene or a sequence with substantial identity to the gene. Other recombination-based methods such as non-homologous recombinations or deletion of endogenous genes by homologous recombination may also be used.

In case of proteins that form heterodimers or other multimers, both or all subunits have to be expressed in one system or cell.

The nucleic acids of the present invention find use as probes for the detection of genetic polymorphisms and as templates for the recombinant production of normal or variant peptides or polypeptides encoded by genes listed in the Examples.

Probes in accordance with the present invention comprise without limitation isolated nucleic acids of about 10-100 bp, preferably 15-75 bp and most preferably 17-25 bp in length, which hybridize at high stringency to one or more of the polymorphic sequences disclosed herein or to a sequence immediately adjacent to a polymorphic position. Furthermore, in some embodiments a full-length gene sequence may be used as a probe. In one series of embodiments, the probes span the polymorphic positions in genes disclosed herein. In another series of embodiments, the probes correspond to sequences immediately adjacent to the polymorphic positions.

## Polymorphic Polypeptides and Polymorphism-Specific Antibodies

The present invention encompasses isolated peptides and polypeptides encoded by genes listed in the Examples comprising polymorphic positions disclosed herein. In one preferred embodiment, the peptides and polypeptides are useful screening targets to identify cardiovascular drugs. In another preferred embodiments, the peptides and polypeptides are capable of eliciting antibodies in a suitable host animal that react specifically with a polypeptide comprising the polymorphic position and distinguish it from other polypeptides having a different sequence at that position.

Polypeptides according to the invention are preferably at least five or more residues in length, preferably at least fifteen residues. Methods for obtaining these polypeptides are described below. Many conventional techniques in protein biochemistry and immunology are used. Such techniques are well known and are explained in Immunochemical Methods in Cell and Molecular Biology, 1987 (Mayer and Waler, eds; Academic Press, London); Scopes, 1987, Protein Purification: Principles and Practice, Second Edition (Springer-Verlag, N.Y.) and Handbook of Experimental Immunology, 1986, Volumes I-IV (Weir and Blackwell eds.).

Nucleic acids comprising protein-coding sequences can be used to direct the ITT recombinant expression of polypeptides encoded by genes disclosed herein in intact cells or in cell-free translation systems. The known genetic code, tailored if desired for more efficient expression in a given host organism, can be used to synthesize oligonucleotides encoding the desired amino acid sequences. The polypeptides may be isolated from human cells, or from heterologous organisms or cells (including, but not limited to, bacteria, fungi, insect, plant, and mammalian cells) into which an appropriate protein-coding sequence has been introduced and expressed. Furthermore, the polypeptides may be part of recombinant fusion proteins.

Peptides and polypeptides may be chemically synthesized by commercially available automated procedures, including, without limitation, exclusive solid phase synthesis, partial solid phase methods, fragment condensation or classical solution synthesis. The polypeptides are preferably prepared by solid phase peptide synthesis as described by Merrifield, 1963, J. Am. Chem. Soc. 85:2149.

Methods for polypeptide purification are well-known in the art, including, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against peptides encoded by genes disclosed herein, can be used as purification reagents. Other purification methods are possible.

The present invention also encompasses derivatives and homologues of the polypeptides. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The present invention also encompasses antibodies that specifically recognize the polymorphic positions of the invention and distinguish a peptide or polypeptide containing a particular polymorphism from one that contains a different sequence at that position. Such polymorphic position-specific antibodies according to the present invention include polyclonal and monoclonal antibodies. The antibodies may be elicited in an animal host by immunization with peptides encoded by genes disclosed herein or may be formed by in vitro immunization of immune cells. The immunogenic components used to elicit the antibodies may be isolated from human cells or produced in recombinant systems. The antibodies may also be produced in recombinant systems programmed with appropriate antibody-encoding DNA. Alternatively, the antibodies may be constructed by biochemical reconstitution of purified heavy and light chains. The antibodies include hybrid antibodies (i.e., containing two sets of heavy chain/light chain combinations, each of which recognizes a different antigen), chimeric antibodies (i.e., in which either the heavy chains, light chains, or both, are fusion proteins), and univalent antibodies (i.e., comprised of a heavy chain/light chain complex bound to the constant region of a second heavy chain). Also included are Fab fragments, including Fab' and F(ab).sub.2 fragments of antibodies. Methods for the production of all of the above types of antibodies and derivatives are well-known in the art and are discussed in more detail below. For example, techniques for producing and processing polyclonal antisera are disclosed in Mayer and Walker, 1987, Immunochemical Methods in Cell and Molecular Biology, (Academic Press, London). The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., Schreier et al., 1980, Hybridoma Techniques; U.S. Pat. Nos. 4,341,761; 4,399,121; 4,427,783; 4,444,887; 4,466,917; 4,472,500; 4,491,632; and 4,493,890. Panels of monoclonal antibodies produced against peptides encoded by genes disclosed herein can be screened for various properties; i.e. for isotype, epitope affinity, etc.

The antibodies of this invention can be purified by standard methods, including but not limited to preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in The Art of Antibody Purification, 1989, Amicon Division, W. R. Grace & Co. General protein purification methods are described in Protein Purification: Principles and Practice, R. K. Scopes, Ed., 1987, Springer-Verlag, New York, N.Y.

Methods for determining the immunogenic capability of the disclosed sequences and the characteristics of the resulting sequence-specific antibodies and immune cells are well-known in the art. For example, antibodies elicited in response to a peptide comprising a particular polymorphic sequence can be tested for their ability to specifically recognize that polymorphic sequence, i.e., to bind differentially to a peptide or polypeptide comprising the polymorphic sequence and thus distinguish it from a similar peptide or polypeptide containing a different sequence at the same position.

**Kits**

As set forth herein, the invention provides diagnostic methods, e.g., for determining the identity of the allelic variants of polymorphic regions present in the gene loci of genes disclosed herein, wherein specific allelic variants of the polymorphic region are associated with cardiovascular diseases. In a preferred embodiment, the diagnostic kit can be used to determine whether a subject is at risk of developing a cardiovascular disease. This information could then be used, e.g., to optimize treatment of such individuals.

In preferred embodiments, the kit comprises a probe or primer which is capable of hybridizing to a gene and thereby identifying whether the gene contains an allelic variant of a polymorphic region which is associated with a risk for cardiovascular disease. The kit preferably further comprises instructions for use in diagnosing a subject as having, or having a predisposition, towards developing a cardiovascular disease. The probe or primers of the kit can be any of the probes or primers described in this file.

Preferred kits for amplifying a region of a gene comprising a polymorphic region of interest comprise one, two or more primers.

**Antibody-based diagnostic methods and kits:**

The invention also provides antibody-based methods for detecting polymorphic patterns in a biological sample. The methods comprise the steps of: (i) contacting a sample with one or more antibody preparations, wherein each of the antibody preparations is specific for a particular polymorphic form of the proteins encoded by genes disclosed herein, under conditions in which a stable antigen-antibody complex can form between the antibody and antigenic components in the sample; and (ii) detecting any antigen-antibody complex formed in step (i) using any suitable means known in the art, wherein the detection of a complex indicates the presence of the particular polymorphic form in the sample.

Typically, immunoassays use either a labelled antibody or a labelled antigenic component (e.g., that competes with the antigen in the sample for binding to the antibody). Suitable labels include without limitation enzyme-based, fluorescent, chemiluminescent, radioactive, or dye molecules. Assays that amplify the signals from the probe are also known, such as, for example, those that utilize biotin and avidin, and enzyme-labelled immunoassays, such as ELISA assays.

The present invention also provides kits suitable for antibody-based diagnostic applications. Diagnostic kits typically include one or more of the following components:

(i) Polymorphism-specific antibodies. The antibodies may be pre-labelled; alternatively, the antibody may be unlabelled and the ingredients for labelling may be included in the kit in separate containers, or a secondary, labelled antibody is provided; and

(ii) Reaction components: The kit may also contain other suitably packaged reagents and materials needed for the particular immunoassay protocol, including solid-phase matrices, if applicable, and standards.

The kits referred to above may include instructions for conducting the test. Furthermore, in preferred embodiments, the diagnostic kits are adaptable to high-throughput and/or automated operation.

**<u>Drug Targets and Screening Methods</u>**

According to the present invention, nucleotide sequences derived from genes disclosed herein and peptide sequences encoded by genes disclosed herein, particularly those that contain one or more polymorphic sequences, comprise useful targets to identify cardiovascular drugs, i.e., compounds that are effective in treating one or more clinical symptoms of cardiovascular disease. Furthermore, especially when a protein is a multimeric protein that are build of two or more subunits, is a combination of different polymorphic subunits very useful.

Drug targets include without limitation (i) isolated nucleic acids derived from the genes disclosed herein, and (ii) isolated peptides and polypeptides encoded by genes disclosed herein, each of which comprises one or more polymorphic positions.

**In vitro screening methods:**

In one series of embodiments, an isolated nucleic acid comprising one or more polymorphic positions is tested in vitro for its ability to bind test compounds in a sequence-specific manner. The methods comprise:

(i) providing a first nucleic acid containing a particular sequence at a polymorphic position and a second nucleic acid whose sequence is identical to that of the first nucleic acid except for a different sequence at the same polymorphic position;

(ii) contacting the nucleic acids with a multiplicity of test compounds under conditions appropriate for binding; and

(iii) identifying those compounds that bind selectively to either the first or second nucleic acid sequence.

Selective binding as used herein refers to any measurable difference in any parameter of binding, such as, e.g., binding affinity, binding capacity, etc.

In another series of embodiments, an isolated peptide or polypeptide comprising one or more polymorphic positions is tested in vitro for its ability to bind test compounds in a sequence-specific manner. The screening methods involve:

(i) providing a first peptide or polypeptide containing a particular sequence at a polymorphic position and a second peptide or polypeptide whose sequence is identical to the first peptide or polypeptide except for a different sequence at the same polymorphic position;

(ii) contacting the polypeptides with a multiplicity of test compounds under conditions appropriate for binding; and

(iii) identifying those compounds that bind selectively to one of the nucleic acid sequences.

In preferred embodiments, high-throughput screening protocols are used to survey a large number of test compounds for their ability to bind the genes or peptides disclosed above in a sequence-specific manner.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

**In vivo screening methods:**

Intact cells or whole animals expressing polymorphic variants of genes disclosed herein can be used in screening methods to identify candidate cardiovascular drugs.

In one series of embodiments, a permanent cell line is established from an individual exhibiting a particular polymorphic pattern. Alternatively, cells (including without limitation mammalian, insect, yeast, or bacterial cells) are programmed to express a gene comprising one or more polymorphic sequences by introduction of appropriate DNA. Identification of candidate compounds can be achieved using any suitable assay, including without limitation (i) assays that measure selective binding of test compounds to particular polymorphic variants of proteins encoded by genes disclosed herein; (ii) assays that measure the ability of a test compound to modify (i.e., inhibit or enhance) a measurable activity or function of proteins encoded by genes disclosed herein; and (iii) assays that measure the ability of a compound to modify (i.e., inhibit or enhance) the transcriptional activity of sequences derived from the promoter (i.e., regulatory) regions of genes disclosed herein.

In another series of embodiments, transgenic animals are created in which (i) one or more human genes disclosed herein, having different sequences at particular polymorphic positions are stably inserted into the genome of the transgenic animal; and/or (ii) the endogenous genes disclosed herein are inactivated and replaced with human genes disclosed herein, having different sequences at particular polymorphic positions. See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for one or more clinical markers of cardiovascular status.

The following are intended as non-limiting examples of the invention.

**Material and Methods**

Genotyping of patient DNA with the Pyrosequencing™ Method as described in the patent application WO 9813523:

First a PCR is set up to amplify the flanking regions around a SNP. Therefor 2 ng of genomic DNA (patient sample) are mixed with a primerset (20 - 40 pmol) producing a 75 to 320 bp PCR fragment with 0,3 to 1 U Qiagens Hot Star Taq Polymerase™ in a total volume of 20 µL. One primer is biotinylated depending on the direction of the sequencing primer. To force the biotinylated primer to be incorporated it is used 0,8 fold.

For primer design, programms like Oligo 6™ (Molecular Biology Insights) or Primer Selects (DNAStar) are used. PCR setup is performed by a BioRobot 3000™ from Qiagen. PCR takes place in T1 or Tgradient Thermocyclers™ from Biometra. The whole PCR reaction is transferred into a PSQ plate ™ (Pyrosequencing) and prepared using the Sample Prep Tool ™ and SNP Reagent Kit ™ from Pyrosequencing according to their instructions.

**Preparation of template for Pyrosequencing™:**

Sample preparation using PSQ 96 Sample Prep Tool:

1. Mount the PSQ 96 Sample Prep Tool Cover onto the PSQ 96 Sample Prep Tool as follows: Place the cover on the desk, retract the 4 attachment rods by separating the handle from the magnetic rod holder, fit the magnetic rods into the holes of the cover plate, push the handle downward until a click is heard. The PSQ 96 Sample Prep Tool is now ready for use.
2. To transfer beads from one plate to another, place the covered tool into the PSQ 96 Plate containing the samples and lower the magnetic rods by separating the handle from the magnetic rod holder. Move the tool up and down a few times then wait for 30-60 seconds. Transfer the beads into a new PSQ 96 plate containing the solution of choice.
3. Release the beads by lifting the magnetic rod holder, bringing it together with the handle. Move the tool up and down a few times to make sure that the beads are released.

All steps are performed at room temperature unless otherwise stated.

Immobilization of PCR product:

Biotinylated PCR products are immobilized on streptavidin-coated Dynabeads™ M-280 Streptavidin. Parallel immobilization of several samples are performed in the PSQ 96 Plate.

1. Mix PCR product, 20 µl of a well optimized PCR, with 25 µl 2X BW-buffer II. Add 60-150 µg Dynabeads. It is also possible to add a mix of Dynabeads and 2X BW-buffer II to the PCR product yielding a final BW-buffer II concentration of approximately 1x.
2. Incubate at 65°C for 15 min agitation constantly to keep the beads dispersed. For optimal immobilization of fragments longer than 300 bp use 30 min incubation time.

*Strand separation:*

4. For strand separation, use the PSQ 96 Sample Prep Tool to transfer the beads with the immobilized sample to a PSQ 96 Plate containing 50 µl 0.50 M NaOH per well. Release the beads.
5. After approximately 1 min, transfer the beads with the immobilized strand to a PSQ 96 Plate containing 99 µl 1x Annealing buffer per well and mix thoroughly.
6. Transfer the beads to a PSQ 96 Plate containing 45 µl of a mix of 1x Annealing buffer and 3-15 pmoles sequencing primer per well.
7. Heat at 80°C for 2 minutes in the PSQ 96 Sample Prep Thermoplate and move to room temperature.
8. After reaching room temperature, continue with the sequencing reaction.

Sequencing reaction:

1. Choose the method to be used ("SNP Method") and enter relevant information in the PSQ 96 Instrument Control software.
2. Place the cartridge and PSQ 96 Plate in the PSQ 96 Instrument.
3. Start the run.

Genotyping with a service contractor:

Qiagen Genomics, formerly Rapigene, is a service contractor for genotyping SNPs in patient samples. Their method is based on a primer extension method where two complementary primers are designed for each genotype that are labeled with different tags. Depending on the genotype only one primer will be elongated together with a certain tag. This tag can be detected with mass spectrometry and is a measure for the respective genotype. The method is described in the following patent: "Detection and identification of nucleic acid molecules - using tags which may be detected by non-fluorescent spectrometry or potentiometry" (WO 9727325).

<u>Examples</u>

Table 1

| Definition of "good" and "bad" serum lipid levels | | |
|---|---|---|
| | "Good" | "Bad" |
| LDL-Cholesterol [mg/dL] | 125 -150 | 170 - 200 |
| Cholesterol [mg/dL] | 190 - 240 | 265 - 315 |
| HDL-Cholesterol [mg/dL] | 60 -105 | 30 - 55 |
| Triglycerides [mg/dL] | 45 - 115 | 170 - 450 |
| Number of Patients | 146 | 132 |

An informed consent was signed by the patients and control people. Blood was taken by a physician according to medical standard procedures.
Samples were collected anonymous and labeled with a patient number.
DNA was extracted using kits from Qiagen.
Table 2a Oligonucleotide primers used for genotyping using mass spectrometry The baySNP number refers to an internal numbering of the CA SNPs. Primer sequences are listed for preamplification of the genomic fragments (primers EF and ER) and for subsequent allele specific PCR of the SNP.

| Primer Name | Primer Sequence | baySNP |
|---|---|---|
| 10600_A251G_EF | CCTGGCAACTAACCTCTT | 10600 |
| 10600_A251G_ER | AGGCAGTCTCTCTGTCTACTC | |
| 10600_A251G_AR | gggacggtcggtagatATTGGCCCTGCTCAGGAT | |
| 10600_A251G_GR | gctggctcggtcaagaATTGGCCCTGCTCAGGAC | |
| | | |
| 4887_A22C_EF | GGACCACACTCACAGACA | 4887 |
| 4887_A22C_ER | GCACTGGGACGTAGCA | |
| 4887_A22C_AF | gggacggtcggtagatCACACTCACAGACAAGTA | |
| 4887_A22C_CF | gctggctcggtcaagaCACACTCACAGACAAGTC | |
| | | |
| 2150_C316T_CR_ | gggacggtcggtagatAACTTTCAAAGGTGATAG | 2150 |
| 2150_C316T_EF_U | GACGATGCCTTCAGCACATAAGAGGCAGTAGAGAAACA | |
| 2150_C316T_ER_ | ATGACAAGCAGAAAGTCC | |
| 2150_C316T_TR_ | gctggctcggtcaagaAACTTTCAAAGGTGATAA | |
| | | |
| 1274_C396G_CR_ | gggacggtcggtagatTCAGCACTTTGGGAGACG | 1274 |
| 1274_C396G_EF_U | GACGATGCCTTCAGCACAGTTGTTGTTGTTTGTTTGG | |
| 1274_C396G_ER_ | GGTAGCTCATGCCTGTAA | |
| 1274_C396G_GR_ | gctggctcggtcaagaTCAGCACTTTGGGAGACC | |
| | | |
| 2281_A227C_AR_ | gggacggtcggtagatCAGGCTTGGAGACCTGGT | 2281 |
| 2281_A227C_CR_ | gctggctcggtcaagaCAGGCTTGGAGACCTGGG | |
| 2281_A227C_EF_U | GACGATGCCTTCAGCACAGGGTATTCAGTTGGAAGG | |
| 2281_A227C_ER_ | AAGGCAAGGTTCTTAGTTG | |

**Table 2b Oligonucleotide primers used for genotyping using Pyrosequencing**

The baySNP number refers to an internal numbering of the CA SNPs. Primer sequences are listed for preamplification of the genomic fragments and for sequencing of the SNP using the pyrosequencing method.

| Primer Name | Primer Sequence | baySNP |
|---|---|---|
| 8039FW | CCTGGAGACATACTCGGCAG | 8039 |
| 8039RE-bio | CAGGAGAAGCACCCACGTGT | |
| 8039-SQ | CTCCTCCTGGCACCCC | |

## Table 3 CA SNPs and putative CA genes

The baySNP number refers to an internal numbering of the CA SNPs. Listed are the different polymorphisms found in our association study. Also accession numbers and descriptions of those gene loci are given that are most homologous to the CA genes as listed in the sequences section (see below). Homologous genes and their accession numbers could be found by those skilled in the art in the Genbank database.

Table 4

| Cohorts | | | | | |
|---|---|---|---|---|---|
| **baySNP** | **GT 11** | **GT 12** | **GT 22** | **Accession** | **DESCRIPTION of putative CA genes** |
| 10600 | AA | AG | GG | AF129756 | Apolipoprotein M |
| 2150 | CC | CT | TT | X03021 | Granulocyte-macrophage colony stimulating factor (GM-CSF) |
| 1274 | CC | CG | GG | M14083 | Beta-migrating plasminogen activator inhibitor I |
| 4887 | AA | AC | CC | M27287 | Oncostatin |
| 2281 | AA | AC | CC | X87872 | Hepatocyte nuclear factor 4 |
| 8039 | CC | CT | TT | Z81010 | Platelet-derived growth factor beta (PDGFB) |

Given are names (as used in table 5) and formations of the various cohorts that were used for genotyping

Table 5

| Cohort sizes and p-values of CA SNPs | |
|---|---|
| **COHORT** | **Definition** |
| HELD_ALL_GOOD/BAD | Healthy elderly individuals of both genders with good or bad serum lipid profiles (as defined in table 1) |
| HELD_FEM_GOOD/BAD | Healthy elderly individuals (female) with good or bad serum lipid profiles (as defined in table 1) |
| HELD_MAL_GOOD/BAD | Healthy elderly individuals (male) with good or bad serum lipid profiles (as defined in table 1) |
| CVD_ALL_CASE/CTRL | Individuals with diagnosis of cardiovascular disease and healthy controls (both genders) |
| CVD_FEM_CASE/CTRL | Individuals with diagnosis of cardiovascular disease and healthy controls (female) |

The baySNP number refers to an internal numbering of the CA SNPs. GTYPE P and A PVAL provide the p values obtained through chi square tests when comparing COHORTS A and B. For GTYPE P (p value of genotypes) the number of patients in cohort A carrying genotypes 11, 12 or 22 (FQ11 A, FQ 12 A, FQ 22 A) were compared with the respective patients in cohort B (FQ11 B, FQ 12 B, FQ 22 B) resulting in a chi square test with a 3×2 matrix. For A PVAL (p value of alleles) we compared the allele count of alleles 1 and 2 in cohorts A and B, respectively (chi square test with a 2×2 matrix). SIZE A and B: Number of patients in cohorts A and B, respectively. See table 4 for definition of COHORTs A and B.

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| **2150** | 0,0075 | 0,4739 | HELD_ALL_BAD | 105 | 7 | 29 | 69 | HELD_ALL_GOOD | 125 | 0 | 44 | 81 |
| **10600** | 0,0276 | 0,0108 | CVD_ALL_CASE | 103 | 0 | 3 | 100 | CVD_ALL_CTRL | 74 | 1 | 8 | 65 |
| **1274** | 0,0271 | 0,0116 | CVD_ALL_CASE | 103 | 0 | 5 | 98 | CVD_ALL_CTRL | 70 | 1 | 10 | 59 |
| **8039** | 0,0132 | 0,0075 | CVD_FEM_CASE | 27 | 9 | 14 | 4 | CVD_FEM_CTRL | 31 | 20 | 11 | 0 |
| **2281** | 0,0179 | 0,0038 | HELD_FEM_CASE | 33 | 6 | 15 | 12 | HELD_FEM_CTRL | 23 | 12 | 8 | 3 |
| **4887** | 0,0261 | 0,0236 | HELD_MAL_CASE | 16 | 0 | 3 | 13 | HELD_MAL_CTRL | 20 | 1 | 11 | 8 |

**Table 6: Correlation of genotypes of CA SNPs to relative risk**

Taking the frequency of genotypes as shown in Table 5 those skilled in the art can calculated a relative risk. If for example relative risk ratio would be 2 the respective genotype of a person would tell that the risk of getting a cardiovascular disease is doubled. The risk is halved if the respective genotype has a ratio of 0.5. In the table below the GG genotype of baySNP-1274 for example would tell that the risk of getting a cardiovascular disease is doubled and the CC genotype of baySNP-4887 would tell that the risk of getting a cardiovascular disease is 3.1 times higher as compared to a non-GG or non-CC genotype, respectively.

Table 6:

| **Correlation of genotypes of CA SNPs to relative risk** | | | | | | |
|---|---|---|---|---|---|---|
| **BAYSNP** | **GTYPE1 1** | **GTYPE1 2** | **GTYPE2 2** | **RR11** | **RR12** | **RR22** |
| **1274** | CC | CG | GG | - | 0,54 | 2 |
| **2150** | CC | CT | TT | 2,28 | 0,82 | 1,02 |
| **2281** | AA | AC | CC | 0,47 | 1,2 | 1,56 |
| **4887** | AA | AC | CC | - | 0,36 | 3,1 |
| **8039** | CC | CT | TT | 0,5 | 1,42 | 2,35 |
| **10600** | AA | AG | GG | - | 0,45 | 2,42 |
| ( - RR cannot be calculated because this particular genotype was not found.) | | | | | | |

For diagnostic conclusions to be drawn from genotyping a particular patient we calculated the relative risk RR1, RR2, RR3 for the three possible genotypes of each SNP. Given the genotype frequencies as

| | gtype1 | gtype2 | gtype3 |
|---|---|---|---|
| case | N11 | N12 | N13 |
| control | N21 | N22 | N23 |

we calculate

$$RR3 = \frac{N13}{N23} \Big/ \frac{N11 + N12}{N21 + N22}$$

$$RR1 = \frac{N11}{N21} \Big/ \frac{N12 + N13}{N22 + N23}$$

$$RR2 = \frac{N12}{N22} \Big/ \frac{N11 + N13}{N21 + N23}$$

Here, the *case* and *control* populations represent any case-control-group pair, or bad(case)-good(control)-group pair, respectively. A value RR1>1, RR2>1, and RR3>1 indicates an increased risk for individuals carrying genotype 1, genotype 2, and genotype 3, respectively. For example, RR1=3 indicates a 3-fold risk of an individual carrying genotype 1 as compared to individuals carrying genotype 2 or 3 (a detailed description of relative risk calculation and statistics can be found in (Biostatistics, L. D. Fisher and G. van Belle, Wiley Interscience 1993)). The baySNP number refers to an internal numbering of the CA SNPs and can be found in the sequence listing.

| BAYSNP | GTYPE1 | GTYPE2 | GTYPE3 | RR1 | RR2 | RR3 | FREQ1_A | FREQ2_A | FREQ3_A | FREQ1_B | FREQ2_B | FREQ3_B |
|---|---|---|---|---|---|---|---|---|---|---|---|---|
| 1274 | CC | CG | GG | 0 | 0,54 | 2 | 0 | 5 | 98 | 1 | 10 | 59 |
| 2150 | CC | CT | TT | 2,28 | 0,82 | 1,02 | 7 | 29 | 69 | 0 | 44 | 81 |
| 4887 | AA | AC | CC | 0 | 0,36 | 3,1 | 0 | 3 | 13 | 1 | 11 | 8 |
| 8039 | CC | CT | TT | 0,5 | 1,42 | 2,35 | 9 | 14 | 4 | 20 | 11 | 0 |
| 10600 | AA | AG | GG | 0 | 0,45 | 2,42 | 0 | 3 | 100 | 1 | 8 | 65 |

## Claims

1. An isolated polynucleotide encoded by a cardiovascular associated (CA) gene; the polynucleotide is selected from the group comprising SEQ ID 1, 2, 3, 4, 5, 6 with allelic variation as indicated in the sequences section contained in a functional surrounding like full length cDNA for CA gene polypeptide and with or without the CA gene promoter sequence.

2. An expression vector containing one or more of the polynucleotides of claim 1.

3. A host cell containing the expression vector of claim 2.

4. A substantially purified CA gene polypeptide encoded by a polynucleotide of claim 1.

5. A method for producing a CA gene polypeptide, wherein the method comprises the following steps:

   a) culturing the host cell of claim 3 under conditions suitable for the expression of the CA gene polypeptide; and
   b) recovering the CA gene polypeptide from the host cell culture.

**6.** A method for the detection of a polynucleotide of claim 1 or a CA gene polypeptide of claim 4 comprising the steps of:

contacting a biological sample with a reagent which specifically interacts with the polynucleotide or the CA gene polypeptide.

**7.** A method of screening for agents which regulate the activity of a CA gene comprising the steps of:

contacting a test compound with a CA gene polypeptide encoded by any polynucleotide of claim 1; and detecting CA gene activity of the polypeptide,

wherein a test compound which increases the CA gene polypeptide activity is identified as a potential therapeutic agent for increasing the activity of the CA gene polypeptide and wherein a test compound which decreases the CA activity of the polypeptide is identified as a potential therapeutic agent for decreasing the activity of the CA gene polypeptide.

**8.** A reagent that modulates the activity of a CA polypeptide or a polynucleotide wherein said reagent is identified by the method of the claim 7.

**9.** A pharmaceutical composition, comprising:

the expression vector of claim 2 or the reagent of claim 8 and a pharmaceutically acceptable carrier.

**10.** Use of the reagent according to claim 8 for the preparation of a medicament.

**11.** A method for determining whether a human subject has, or is at risk of developing a cardiovascular disease, comprising determining the identity of nucleotide variations as indicated in the sequences section of SEQ ID 1, 2, 3, 4, 5, 6 of the CA gene locus of the subject; whereas a "risk" genotype has a risk ratio of greater than 1 as can be seen from table 6.

**12.** A kit for assessing cardiovascular status, said kit comprising

a) sequence determination primers and
b) sequence determination reagents,

wherein said primers are selected from the group comprising primers that hybridize to polymorphic positions in human CA genes according to claim 1; and primers that hybridize immediately adjacent to polymorphic positions in human CA genes according to claim 1.

**13.** A kit as defined in claims 12 detecting a combination of two or more, up to all, polymorphic sites selected from the groups of sequences as defined in claim 1.

**14.** A kit for assessing cardiovascular status, said kit comprising one or more antibodies specific for a polymorphic position defined in claim 1 within the human CA gene polypeptides and combinations of any of the foregoing.

European Patent Office

# PARTIAL EUROPEAN SEARCH REPORT

which under Rule 45 of the European Patent Convention shall be considered, for the purposes of subsequent proceedings, as the European search report

Application Number EP 02 00 0253

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/001823 A1 (CAIRNS WILLIAM JOHN ET AL) 3 January 2002 (2002-01-03)<br>* page 1, paragraph 4 * | 1-7 | |
| Y | WO 98 40517 A (MEDICAL SCIENCE SYS INC) 17 September 1998 (1998-09-17)<br>* page 1-14; claims 1,4,7,10,14-25 * | 1-7,9, 11-14 | |
| Y | WO 99 14363 A (EURONA MEDICAL AB ;KARPE FREDRIK (SE)) 25 March 1999 (1999-03-25)<br>* page 2, line 23 - page 3, line 24; claims 5,6,10-12 * | 1-7,9, 11-14 | |
| Y | CAMBIEN F ET AL: "SEQUENCE DIVERSITY IN 36 CANDIDATE GENES FOR CARDIOVASCULAR DISORDERS"<br>AMERICAN JOURNAL OF HUMAN GENETICS, UNIVERSITY OF CHICAGO PRESS, CHICAGO,, US, vol. 65, no. 1, July 1999 (1999-07), pages 183-191, XP000920770<br>ISSN: 0002-9297<br>* the whole document * | 1-7,9, 11-14 | |
| | -/-- | | |

TECHNICAL FIELDS SEARCHED (Int.Cl.7)

C07K
A61K
C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 29 November 2002 | Marchesini, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

## INCOMPLETE SEARCH SHEET C

Application Number
EP 02 00 0253

Claim(s) searched completely:
1-7, 11-14

Claim(s) searched incompletely:
9

Claim(s) not searched:
8, 10

Reason for the limitation of the search:

Claims 8, 9 (partially) and 10 refer to a reagent without indicating any technical features of said reagent.

The reagent is solely defined by reference to a desirable characteristic or property, namely its abilty to modulate the activity of a CA polypeptide of the present application.

Consequently, said claims are not supported by the description as required by Art. 84 EPC and the application does not provide disclosure within the meaning of Art. 83 EPC for such compounds.

European Patent Office

# PARTIAL EUROPEAN SEARCH REPORT

Application Number
EP 02 00 0253

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | HERTZ RACHEL ET AL: "Fatty acyl-CoA thioesters are ligands of hepatic nuclear factor-4alpha." NATURE (LONDON), vol. 392, no. 6675, 2 April 1998 (1998-04-02), pages 512-516, XP002193639 ISSN: 0028-0836 * abstract * * page 515, column 1, last paragraph * | 1-7,9, 11-14 | |
| Y,D | HWANG DAVID M ET AL: "A genome-based resource for molecular cardiovascular medicine: Toward a compendium of cardiovascular genes." CIRCULATION, vol. 96, no. 12, 16 December 1997 (1997-12-16), pages 4146-4203, XP001068303 ISSN: 0009-7322 * table 9 * Appendix I | 1-7,9, 11-14 | TECHNICAL FIELDS SEARCHED (Int.Cl.7) |
| Y | OSSEI-GERNING N ET AL: "PLASMINOGEN ACTIVATOR INHIBITOR-1 PROMOTER 4G/5G GENOTYPE AND PLASMA LEVELS IN RELATION TO A HISTORY OF MYOCARDIAL INFARCTION IN PATIENTS CHARACTERIZED BY CORONARY ANGIOGRAPHY" ARTERIOSCLEROSIS, THROMBOSIS, AND VASCULAR BIOLOGY, XX, XX, vol. 17, no. 1, 1997, pages 33-37, XP002041862 ISSN: 1079-5642 * the whole document * | 1-7,9, 11-14 | |

-/--

EPO FORM 1503 03.82 (P04C10)

European Patent Office

# PARTIAL EUROPEAN SEARCH REPORT

Application Number
EP 02 00 0253

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| Y | WO 01 87907 A (DUDA AMY E ;KAZEMI AMIR (US); KOSHY BEENA (US); GENAISSANCE PHARMA) 22 November 2001 (2001-11-22)<br>* page 1 - page 7, line 6; figure 1 *<br>--- | 1-7,9, 11-14 | |
| Y | EP 0 485 961 A (BRISTOL MYERS CO) 20 May 1992 (1992-05-20)<br>* page 8, line 32 - page 8, line 45 *<br>--- | 1-7,9, 11-14 | |
| A | PANG C-P: "MOLECULAR DIAGNOSTICS FOR CARDIOVASCULAR DISEASE" CLINICAL CHEMISTRY AND LABORATORY MEDICINE, WALTER DE GRUYTER UND CO, DE, vol. 36, no. 8, 1998, pages 605-614, XP001062847<br>ISSN: 1434-6621<br>* the whole document *<br>--- | 1-7,9, 11-14 | TECHNICAL FIELDS SEARCHED (Int.Cl.7) |
| A | WO 99 57306 A (HEATON JEREMY P W ;BRIEN SUSAN E (CA); UNIV KINGSTON (CA); ADAMS M) 11 November 1999 (1999-11-11)<br>* abstract; claims 18-21 *<br>--- | 1-7,9, 11-14 | |
| A | TAKATA YASUNORI ET AL: "Novel cis element for tissue-specific transcription of rat platelet-derived growth factor beta-receptor gene." HYPERTENSION (BALTIMORE), vol. 33, no. 1 PART 2, January 1999 (1999-01), pages 298-302, XP002220213<br>ISSN: 0194-911X<br>* abstract *<br>---<br>-/-- | 1-7,9, 11-14 | |

EPO FORM 1503 03.82 (P04C10)

European Patent Office

# PARTIAL EUROPEAN SEARCH REPORT

Application Number
EP 02 00 0253

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | DUAN A J ET AL: "Proposed lipocalin fold for apolipoprotein M based on bioinformatics and site-directed mutagenesis" FEBS LETTERS, ELSEVIER SCIENCE PUBLISHERS, AMSTERDAM, NL, vol. 499, no. 1-2, 15 June 2001 (2001-06-15), pages 127-132, XP004247001 ISSN: 0014-5793 * page 1 * --- | 1-7,9, 11-14 | |
| A | WO 94 04196 A (IMP CANCER RES TECH ;VILE RICHARD GEOFFREY (GB); HART IAN ROGER (G) 3 March 1994 (1994-03-03) * page 11, line 25 - page 12, line 30 * --- | 1-7,9, 11-14 | TECHNICAL FIELDS SEARCHED (Int.Cl.7) |
| A | WO 01 66764 A (MEYERS RACHEL A ;MILLENNIUM PHARM INC (US)) 13 September 2001 (2001-09-13) * page 15, line 27 - page 16, line 22 * * page 17, line 17 - page 19, line 6 * * claims 1,3-5,12-16 * --- | 1-7,9, 11-14 | |
| A | DATABASE GENESEQ 'Online! retrieved from FASTA Database accession no. AAQ58039 XP002220214 ----- | 1-7,9, 11-14 | |

EPO FORM 1503 03.82 (P04C10)

European Patent Office

Application Number
EP 02 00 0253

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 02 00 0253

The Search Division considers that the present European patent application does not comply with the requirements of unity of invention and relates to several inventions or groups of inventions, namely:

1. Claims: 1-14 (partly)

   Relates to the isolated polynucleotide (SEQ ID 1) encoded by a cardiovascular associated gene, namely hepatocyte nuclear factor 4.
   This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

2. Claims: 1-14 (partly)

   Relates to the isolated polynucleotide (SEQ ID 2) encoded by a cardiovascular associated gene, namely Beta-migrating plasminogen activator inhibitor I.
   This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

3. Claims: 1-14 (partly)

   Relates to the isolated polynucleotide (SEQ ID 3) encoded by a cardiovascular associated gene, namely Platelet-derived growth factor beta (PDGFB).
   This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

4. Claims: 1-14 (partly)

   Relates to the isolated polynucleotide (SEQ ID 4) encoded by

European Patent Office

LACK OF UNITY OF INVENTION
SHEET B

Application Number
EP 02 00 0253

The Search Division considers that the present European patent application does not comply with the requirements of unity of invention and relates to several inventions or groups of inventions, namely:

a cardiovascular associated gene, namely Apolipoprotein M. This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

5. Claims: 1-14 (partly)

Relates to the isolated polynucleotide (SEQ ID 5) encoded by a cardiovascular associated gene, namely Oncostatin. This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

6. Claims: 1-14 (partly)

Relates to the isolated polynucleotide (SEQ ID 6) encoded by a cardiovascular associated gene, namely Granulocyte-macrophage colony stimulating factor (GM-CSF). This invention further relates to an expression vector containing this polynucleotide, the polypeptide encoded by this polynucleotide, a method for producing the polypeptide, a screening method for agents which regulate the activity of the polypeptide, a reagent that modulates the activity of the polypeptide, a pharmaceutical composition containing the expression vector with this polynucleotide, a diagnostic method, the use of this polynucleotide in a kit for assessing cardiovascular status.

## ANNEX TO THE EUROPEAN SEARCH REPORT ON EUROPEAN PATENT APPLICATION NO. EP 02 00 0253

This annex lists the patent family membersrelating to the patent documents cited in the above-mentioned European search report.
*The members are as contained in the European Patent Office EDP file on*
The European Patent Office is in no way liable for these particulars which are merely given for the purpose of information.

29-11-2002

| Patent document cited in search report | | Publication date | Patent family member(s) | | | Publication date |
|---|---|---|---|---|---|---|
| US 2002001823 | A1 | 03-01-2002 | WO | 0229046 | A1 | 11-04-2002 |
| | | | EP | 1210431 | A1 | 05-06-2002 |
| WO 9840517 | A | 17-09-1998 | US | 6210877 | B1 | 03-04-2001 |
| | | | AU | 738875 | B2 | 27-09-2001 |
| | | | AU | 6457498 | A | 29-09-1998 |
| | | | BR | 9808313 | A | 16-05-2000 |
| | | | CN | 1255948 | T | 07-06-2000 |
| | | | DE | 983381 | T1 | 09-11-2000 |
| | | | EP | 0983381 | A1 | 08-03-2000 |
| | | | ES | 2156843 | T1 | 01-08-2001 |
| | | | GR | 2001300016 | T1 | 30-04-2001 |
| | | | JP | 2001514522 | T | 11-09-2001 |
| | | | NO | 994365 | A | 09-11-1999 |
| | | | NZ | 337642 | A | 23-02-2001 |
| | | | PL | 335603 | A1 | 08-05-2000 |
| | | | WO | 9840517 | A1 | 17-09-1998 |
| | | | ZA | 9801937 | A | 06-09-1999 |
| WO 9914363 | A | 25-03-1999 | AU | 8881498 | A | 05-04-1999 |
| | | | EP | 1015631 | A1 | 05-07-2000 |
| | | | WO | 9914363 | A1 | 25-03-1999 |
| | | | JP | 2001516592 | T | 02-10-2001 |
| | | | US | 6218524 | B1 | 17-04-2001 |
| WO 0187907 | A | 22-11-2001 | AU | 6468101 | A | 26-11-2001 |
| | | | WO | 0187907 | A2 | 22-11-2001 |
| EP 0485961 | A | 20-05-1992 | CA | 2055122 | A1 | 14-05-1992 |
| | | | EP | 0485961 | A1 | 20-05-1992 |
| | | | JP | 4273825 | A | 30-09-1992 |
| WO 9957306 | A | 11-11-1999 | AU | 3590199 | A | 23-11-1999 |
| | | | CA | 2330628 | A1 | 11-11-1999 |
| | | | WO | 9957306 | A2 | 11-11-1999 |
| | | | US | 2002106634 | A1 | 08-08-2002 |
| | | | US | 6376169 | B1 | 23-04-2002 |
| WO 9404196 | A | 03-03-1994 | WO | 9404196 | A1 | 03-03-1994 |
| WO 0166764 | A | 13-09-2001 | AU | 4344701 | A | 17-09-2001 |
| | | | WO | 0166764 | A2 | 13-09-2001 |
| | | | US | 2002098577 | A1 | 25-07-2002 |

EPO FORM P0459

For more details about this annex : see Official Journal of the European Patent Office, No. 12/82

SEQUENCE LISTING

<110> Bayer AG

<120> Genetic polymorphisms predicting Cardiovascular Disease and Medication Efficacy

<130> Empty

<160> 6

<170> PatentIn version 3.1

<210> 1

<211> 859

<212> DNA

<213> Homo Sapiens

<220>

<221> variation

<222> (227)..(227)

<223> BaySNP:2281, A227C

<400> 1
aatctgtgag gctcaccagt caggcacacc aactcaagac tgaacaacaa aattagtgct 60

gcaaagaagc agagcgtctc cctggctgag ccgtggagcc acagtgccca gtgctgggta 120

ttcagttgga aggtgccagt cacaggggct gcagtgtcaa acaggtgttc agagcactga 180

cctcaggctg cattggagag gactgatcct gtttgcatac cacaaamcca ggtctccaag 240

cctgctcagc aatgcatgag tcacctggaa ttctttgaat atccttttca atatccattc 300

tttgaacatc cgtttcttta taagttaacc ggagctggtt tatgttacct gcaactaaga 360

accttgcctt atatagaagt tggtgtcagg agtggttaca ggcagtggat gttggaggaa 420

gctcggagaa gctggaattg gttatctgga ctgcttgggg ctgaagtcag taaaaattca 480

gcacatcata agggctcaga tctggaagcc atggcatgca ggggaagcag aatgtcagat 540

ttgccacctg gagtcaccgg gaaggaagag cccgttgaaa acaaagcttc attgagaatt 600
cattgcctta gaaaagaatg aaaggaatga ggaattagac tgaatgggct ctacagcact 660
gcactgttaa agatgcagaa gcccgtctcc taaattccta aattcttggc tcagacccag 720
atcaaaaact gagacttctg ggccaggtgc agtacctcat gcctgtaatc ccggtacttt 780
gcgaggccga gcaggaggat cacttttgct cagagtccga gaccacctgg gcacatatga 840
gaccccatct ttactagta 859

<210> 2
<211> 461
<212> DNA
<213> Homo Sapiens

<220>
<221> variation
<222> (396)..(396)
<223> BaySNP:1274, C396G

<400> 2
gaattcattg aatgtatggg agcaaaattt gaagactttg aaatctatgt gtcctgccat 60
gttctggaga ggtgagactt gtgtttgata agacactgtg tccagagtgg gtaaattgcc 120
tatgagtctg tagtattttg ttgttgttgt tgtttgtttg gtttttgaga caggctgtgt 180
catctatgct ggagtgcagt ggtgcaatca cagctcacta cagtcttgat ctcccaggat 240
caagcaattc tcttacctca atctcccaat tagctgagac cacaggtgtg tgccaccaca 300
ccaggctaat taaaaaaaaa atttgtgaat atgaagtctt gctatgttgc ccaggctggt 360
cttgaactcc tgggctcatg caaccctcct gccttsgtct cccaaagtgc tgaggttaca 420
ggcatgagct accatgccca gctgatcctt catttgaatt c 461

<210> 3
<211> 872
<212> DNA
<213> Homo Sapiens

<220>
<221> variation

<222> (558)..(558)

<223> BaySNP8039, C558T

<400> 3
ctggggcccc ctcctgcctg cctcaccgcc ccccagcaca ggcgggattg aaagggcccg 60

ggaatgcttt tgagagggag ccaggggccc aacggggaga ggaaacaaag gcaggaaatg 120

ctgttccccc gtagatagcg tctgggcaag gattacaaag tgacaaagag acagaacccc 180

agagggaagg ggacctgggg gtgcaggggg gctgctgacc agggctccag ggaacaggcc 240

agaggttaga atagaatgga atttgctctg aagacagcgt ttataaatac attgtcaatc 300

cagcccgcca gccccgccac gtgtgtgcca ccgagcacgt gcccattcgc tgagtgagct 360

ctggggcacg ggcagggctg cctgctggtc gaggccaggt gtccccaggg gacctcacag 420

accgggcccc tagccagagg ggcggggaga tgactcctcc agtgccaccc tccctggctg 480

ggcccacacc tggagacata ctcggcagat gccctggcac cttctccccg ccctccctcc 540

tcctggcacc ccaccctygg caccaagccg gagccacacg tgggtgcttc tcctgccaca 600

ccccaagtcc caggtaccaa cccgcctgct gactcggccc tgccctggat gggtgtggcc 660

acgctctctc tgggctgcat gggcctctag ttctccagac gtcaagaaga aaggatgcct 720

cctgtcctgc cctcccaaat ttttaaagtc tcctcggagg gcccggagcg cggggcgagg 780

attccattac ctccggggtc tcggtgcagc agcgttggag atataaagag cgatcgagtg 840

gtactcacat ttataagctc tcggatgggt cc 872

<210> 4

<211> 597

<212> DNA

<213> Homo Sapiens

<220>

<221> variation

<222> (510)..(510)

<223> baySNP: 10600, A510G

<400> 4
gggaagggaa aggacctgcc agtgggcagg gtccttaagg aggccatctg ttttggcagg 60

atacgtacta tcagccccgt gccctggaga aacatgctga cagcatcctg gcactggtat 120

gtctaccctg gcatctggga ctctgcatct ttcagaccca cctgtcctct tgaacttagc 180

cctccctaac ttgggagcaa cagtgccctg gggttggggg atgccctggg ctctgcagca 240
gacctctcca acacagacac acaggcacac tctaaatgtg catacttggc actccccttt 300
ggtatgtagc actgctttct ctgggggcaa cctgagccgt aaggaaaaca tgtttactct 360
tgggaccatc tgcaggtgct aagtcctgca gttccccagt gacctgtcct ccctggcaac 420
taacctcttt ggctgcaggg acagctgttc tttctggcac agttcctgtg gtgacgtttg 480
cgcatctctt ggggaccagg ccaggggctr tcctgagcag ggccaattga aaaggcttga 540
ttaacttgag tagacagaga gactgccttt aaatgtgaga gaattatgtc tggagat 597

<210> 5
<211> 936
<212> DNA
<213> Homo Sapiens

<220>
<221> variation
<222> (22)..(22)
<223> baySNP: 4887, A22C

<400> 5
ggaccacact cacagacaag tmagccttga aatgctagac ctcatgcacc ccaatccaat 60
ctcccactgg acagatccgg aaactgaggc ccgagaggga agaggacccc aagatcactg 120
caggcaaggt cggagtgatg ttggggggct ggtgggatgc ccgtggggaa tgaagagctt 180
caagagggct ggactctgtc ttgctcactg ctacgtccca gtgcctagac cagagcctgg 240
caggtgtgca ggaaacacac tgagtaagca ggtgagcatg gtgatggtgt ccggagtccc 300
gccccgggac cacagagcta ggaaagctgg agggccagcc ttagctcctc agggttaagg 360
ctggaataac atagcgactc agttccccga cctggccata tggagtgggg ctaggcctgt 420
cctccctgct tctcactccc ttcctaaccc catagttctc tgagactcag atgccacaag 480
ggcccaggtg cttacatagg ggtccaggag tctgctggtg tcctgcatga gatctgtctg 540
cttctggagc tggccaagga gcacgcggta ctctttcgag cagctgccta tagccgccat 600
gctcgccatg cttggaaaca ggagtgcaag gaccagacct aaggcagaga agaggggtgt 660
cacctgactt ggtgaggaaa gccacagatg ggagcctcgg ggaggggggtt caagagggcc 720
ttgaaaggtg cctccttcat cccagacttt gtgtagtgga gggcgggggc tgggcactgt 780
gtggcacggt gtgtgcccag gtgtgcccac caaggccacc atcatctccc cagtaagtca 840
ctgcccaggg cctgacacag caagcagtgg gagggactga gacagtggct gcgatttacc 900

aagtaggtct ttgagcaaag gctgcagtgc ctggcc 936

<210> 6

<211> 570

<212> DNA

<213> Homo Sapiens

<220>

<221> variation

<222> (316)..(316)

<223> BaySNP:2150, C316T

<400> 6
ccctgctctg ccctgggacc aaaaaggcag gcgtttgact gcccagaagg ccaacctcag 60

gctggcactt aagtcaggcc cttgactctg gctgccactg gcagagctat gcactccttg 120

gggaacacgt gggtggcagc agcgtcacct gacccaggtc agtgggtgtg tcctggagtg 180

ggcctcctgg cctctgagtt ctaagaggca gtagagaaac atgctggtgc ttccttcccc 240

cacgttaccc acttgcctgg actcaagtgt tttttatttt tctttttta aaggaaactt 300

cctgtgcaac ccagaytatc acctttgaaa gtttcaaaga gaacctgaag gactttctgc 360

ttgtcatccc ctttgactgc tgggagccag tccaggagtg agaccggcca gatgaggctg 420

gccaagccgg ggagctgctc tctcatgaaa caagagctag aaactcagga tggtcatctt 480

ggagggacca aggggtgggc cacagccatg gtgggagtgg cctggacctg ccctgggcca 540

cactgaccct gatacaggca tggcagaaga 570